# EUROPEAN PATENT APPLICATION

(11) **EP 4 699 568 A2**
(43) Date of publication of application: **25.02.2026**
(21) Application number: 26151111.7
(22) Date of filing: 29.04.2022
(51) Int. Cl.: A61C 17/02

(54) **DENTAL APPARATUS AND METHOD**

(30) Priority: 18.05.2021 EP 21174535
(62) Divisional of application: 22726701.0
(71) Applicant: Odne AG, 8600 Dübendorf (CH)
(72) Inventor: SCHNEIDER, Andreas, 8003 Zürich (CH); BABKEVICH, Peter, 5430 Wettingen (CH); SCHMOCKER, Andreas, 1018 Lausanne (CH); BISPINGHOFF, Mark, 8051 Zürich (CH)
(74) Representative: GatesIP

(57) **Abstract**

A needle assembly for an endodontic apparatus, comprising: a head for removably coupling the needle assembly to a handpiece; and a needle extending from the head at a proximal end to a distal tip, a lumen of the needle extending to an opening at the tip to deliver fluid into a root canal, wherein the needle tip is positionable within a portion of the root canal; and the lumen has a diameter to enable fluid to be delivered at a delivery pressure in excess of a threshold cavitation pressure, wherein delivery of fluid through the needle at a delivery pressure in excess of a threshold cavitation pressure into the root canal generates a backflow in the root canal that facilitates forming of a cloud of inertial cavitation within the fluid in the root canal forward of the needle tip.

## Description

### Field of Invention

The present invention relates to a dental apparatus and method, particularly an endodontic apparatus and method for endodontic debridement, irrigation, and/or disinfection.

### Background

Root canal treatment is used to preserve teeth in the event of severe infection. A typical root canal treatment procedure involves the steps of: (i) opening the cavity and accessing the pulp and the root canal; (ii) using mechanical instrumentation and enlargement with files (iii) chemical irrigation with sodium hypochlorite (bleach, NaOCl), EDTA and/or other chemical agents (using a syringe), typically repeated several times; (iv) optional activation of NaOCl/chemical agents with ultrasonic cleaning device and (iv) obturation (or filling) of the root canal and closing the tooth. Such multi-step procedures are very laborious and can take around 60 min to complete.

Whilst such procedures are commonly performed (around 55 million procedures per year globally) they are laborious and time consuming and have a relatively high failure rate (approximately 30% of cases). This results in in costly and complicated re-treatments, tooth extractions, or expensive implants being placed. The main causes of such failures are incomplete bacteria removal or weakened tooth structure due to mechanical filing. The former is the result of limitations of the combination of mechanical files and syringe irrigation that cannot access all of the very small (for example less than 300 or 100 µm) and complex canal structure of the tooth, leaving bacteria behind and causing reinfection later. The latter comes about from the enlargement of the canals in order to provide better access for NaOCl. This weakens the dentine structure and makes the root more susceptible to cracks.

Some of the common problems faced by a dentist when performing a root canal procedure include one or more of: substantial time and labour required to enlarge canals; risk of weakening of the tooth structure through filing of canals; risk of failure to remove bacteria in smallest of canals to avoid reinfection through small canals, either not being found and/or by the irrigant not reaching the canals; the occurrence and/or detection of vapour lock within the canal (when air is trapped and unable to escape and pass the liquid above, blocking the irrigant from disinfecting the apical third of the canal); the risk of NaOCl extruding past the apex into the soft and hard tissues; and/or blood inflow from outside the tooth.

Several systems are commercially available that seek to provide improved irrigation of root canals and address or mitigate at least some of the above problems. Such systems are intended to provide enhanced hydrodynamic action in the irrigant to provide improved cleaning debridement and/or disinfection.

One such system is those which use negative pressure suction to prevent the development of vapour lock, an example of which is the EndoVac system (available from Discus Dental of Culver City, CA, USA). Such a system uses continuous apical negative-pressure irrigation in which fresh irrigant is slowly injected at the top into the pulp chamber with a large needle. This is suctioned into the cannula at the bottom of the canal. An issue with such systems is that they require substantial instrumentation to enlarge canals sufficiently to fit two irrigation needles.

A further type of system are sonic or ultrasonic systems which use a vibrating plastic or metal tip that is inserted into the root canal. Typically, the vibration frequency is between 100 Hz and 20 kHz. The vibrations prevent the formation of an air-lock and can improve debridement and/or disinfection performance, particularly in larger canals. Examples of such systems include the EDDY system (available from VDW of Munich, Germany), the Endoactivator system (available from Dentsply Sirona of York, PA, USA), and the Irrisafe system (available from the Acteon Group of Norwich, UK). However, such systems generally only work to a very limited extend without significant prior instrumentation and filing of the canal.

A further type of system are closed hydrodynamic activation systems including the Gentlewave system (available from Sonendo, Inc of Laguna Hills, CA, USA) and the system disclosed in US Patent 4,993,947. In a closed system a part, such as a coronal head, is positioned in the pulp chamber and tightly attached to form a closed system with the tooth. The requirement for a fluid tight connection between the tooth and system requires additional work as part of the dental procedure. A pump supplies alternating flow in and out of the root canal that gradually removes debris. To enhance the hydrodynamic action of the irrigant within the tooth canals US 4,993,947 passes the fluid through a venturi nozzle generating cavitation prior to delivery into the tooth irrigate the entire volume of the root canals (including narrow canals) and increase penetration into the root tissue. In the Gentlewave system (available from Sonendo, Inc of Laguna Hills, CA, USA) the enhancement of the hydrodynamic action comes from the interaction of the incoming water jet striking a platform formed as part of the corona head. However, beside the major disadvantage of creating a cumbersome and tight fixation on the tooth, alternating flow systems have shown significant issues with negative pressure (or insufficiently high) pressure at the apex which then led to blood inflow and inefficient disinfection and debridement in the apex area.

Open hydrodynamic activation systems have also been proposed, for example the RinsEndo system (available from Duerr-Dental of Bittigheim-Bissingen, Germany) and the systems shown in US Patents 4,247,288 and 6,224,378. The RinsEndo system for example uses a special disposable cannula intended to enable irrigant to be delivered more deeply into the root canal. US 6,224,378 uses a hydrojet of high pressure, high velocity water or other liquid which is intended to remove soft tissue within the tooth but have limited ability to cut or erode the hard calcified tooth tissue.

Finally, laser activation systems such as Photon Induced Photoacoustic Streaming (PIPS) or SWEEPS (Shock Wave Enhanced Emission Photo-acoustic Streaming) (available from Fotona of Ljubljana, Slovenia) can use the insertion of an optical fibre system inside the pulp chamber to enhance the irrigation. In such systems a laser is used to superheat water to generate cavitation.

Whilst many root canal activation systems claim to generate 'cavitation' to ensure efficient root canal disinfection, the applicant has found that this is not sufficient in practice. For example, in many systems the cavitation generated is limited and appears to be non-inertial cavitation in which bubbles in the fluid merely oscillate in size and/or shape. Non-inertial cavitation does not cause collapsing bubbles which result in powerful shockwaves as seen in inertial cavitation. Further, the applicant has also found that many of the existing solutions are unable to generate cavitation within the thin canal portions of the tooth (for dimensions smaller than 1 mm, smaller than 500µm or even below 300 or 100µm) that is required for effective cleaning, debridement and/or disinfection. Finally, none of the existing devices can be used to remove or reduce the need for mechanical filing, which is the primary cause for root fractures. They only act as irrigant activation devices to improve the disinfection power of the irrigant.

Thus, there remains a need for an improved apparatus and method for providing fast and reliable cleaning of root canals of necrotic tissue, debris, and bacterial biofilms. In particular, there is a need for apparatus and methods which remove or reduce the need for mechanical filing, and which may, for example, greatly reduce the treatment time required. Embodiments of the invention address these needs and overcome at least some of the known problems of root canal procedures.

### Summary of Invention

According to an aspect of the invention, there is provided a needle assembly for an endodontic apparatus, comprising: a head for removably coupling the needle assembly to a handpiece; and a needle extending from the head at a proximal end to a distal tip, a lumen of the needle extending to an opening at the tip to deliver fluid into a root canal, the opening at the tip of the needle being a forward-facing axial opening such that the fluid is expelled in a forward axial stream from the lumen; wherein at least the distal end of the needle has a needle gauge size between 30G and 34G, such that the needle tip is positionable within a portion of the root canal to enable a backflow to pass between the root canal and the needle; and the lumen has a diameter at the tip of at least 50 µm to enable fluid to be delivered at a delivery pressure in excess of a threshold cavitation pressure, wherein delivery of fluid through the needle at a delivery pressure in excess of a threshold cavitation pressure into the root canal generates a backflow in the root canal that facilitates forming of a cloud of inertial cavitation within the fluid in the root canal forward of the needle tip.

The needle may have a needle gauge between 30G and 34G according to the Birmingham gauge system. The needle gauge may be specified and measured at the needle tip such that the distal end of the needle has a needle gauge between 30G and 34G.

The applicant has recognised that in order to provide effective debridement and/or disinfection it is important that inertial cavitation occurs within the root canal. For such cavitation to occur, the needle tip must have sufficient length and must have a sufficiently large inner diameter. A needle with a needle gauge size between 30G and 34G is small enough to enter at least the coronal part of the root canal which contrasts with many prior art devices where the needle is merely inserted into the pulp chamber.

Without being bound by any specific theory, the applicant considers that in order to produce a cavitation cloud which will effectively clean the root canal and to avoid substantial outflow at the apex, it is desirable to produce a backflow within the root canal - such a backflow requires the needle to provide sufficient clearance in the root canal to allow the exiting flow to pass between the sides of the needle and the sides of the root canal. A backflow provides a significant shear layer within the irrigant in the root canal. The shear layer results in strong vortices being generated at the interface between inward and outward flow inside the root canal. Inside the vortex, the (dynamic) pressure is strongly reduced and such a reduction in pressure will make cavitation more favourable. In addition, the cavitation does not only debride, irrigate, and disinfect the canal, but the flow acts as a highly efficient mechanism to remove any debris or bacteria.

Once the needle is positioned within the root canal (in such a manner to allow the creation of a backflow) the specific pressure required to generate a cloud of inertial cavitation can be selected based upon the specific needle and canal geometry. Such threshold pressures can, for example, be determined for a variety of needle sizes.

The applicant has also identified that providing a lumen having a diameter at the tip of at least 50 µm helps ensure sufficient flow at the needle tip without being limited by frictional pressure loss as the irrigant passes through the lumen of the needle. The selection of a delivery pressure above a threshold cavitation pressure (for example based on for the specific needle and root geometry) may ensure that the flow of irrigant exiting the needle has a velocity which causes a cloud of inertial cavitation to be formed within the irrigant fluid forward of the nozzle tip.

A pump may be provided, which may be a mechanical pump or may be a compressed gas pressurisation system. The pump may provide a delivery pressure of between 5 and 300 bar. For example, the delivery pressure may be between 5 and 100 bar. The applicant has found that, depending upon the needle size and length selected, threshold cavitation pressures in embodiments may be between 5 and 80 bar.

The needle length may be at least 5mm. The length may for example be between 10 and 30mm (for example the needle length may be between 10mm and 20mm). The selection of an appropriate needle length may balance the requirements of ensuring that the tip can be positioned appropriately within the root canal and that frictional flow losses in the needle are acceptable.

The delivery pressure may be selected such that the minimum exit velocity of the irrigant at the needle tip is at least 20 m/s (and for example at least 40 m/s, 60 m/s, 80 m/s, 100 m/s, 150 /s, 200 m/s, or 300 m/s). The flow rate of irrigant through the needle is below 175ml/min (and for example less than 75 ml/min, 50 ml/min, 25 ml/min, 10 ml/min, or 5 ml/min). In contrast, prior art systems may rinse canals using flows of NaOCl at flows as high as 1ml/s which provided much greater risk of inducing pain or damage than with embodiments of the invention.

It will be appreciated that the dimensions of the needle (i.e. the external diameter and/or the lumen diameter) are generally defined based upon the properties at the needle tip (as they may not be constant along the length of the needle).

As embodiments of the invention utilise the hydrodynamic effects of the cavitation cloud to provide debridement and/or disinfection action, it is not be necessary to utilise chemical disinfection agents such as NaOCl for irrigation. As such, advantageously embodiments of the invention use water or saline solution as the irrigant fluid. Saline solution, particularly physiological saline solution (for example 0.9 % NaCl), is generally better tolerated by the body in the event of extrusion beyond the apex and is less likely to cause significant pain or discomfort to the patient than chemical disinfectants such as NaOCl.

In embodiments, the size of the cavitation cloud extends to 1mm, 3mm, 5mm, 7mm, 10mm, 15mm, or 20mm beyond the distal tip of the needle, and its position and size are adapted by changing the needle size, exit speed and/or flow rate.

In embodiments, in order to navigate the complex canals, the needle should be flexible, and laterally bendable.

The irrigant fluid, for example, saline, may include one or more additives. For example, the irrigant fluid may further comprise a disinfecting agent. The disinfecting agent may enhance the bactericidal effect of the irrigant. The irrigant could be a low surface tension liquid such that cavitation occurs more readily at a lower pressure and/or temperature, for example ethanol could be used as a low surface tension liquid which is also a disinfecting agent. The viscosity of the liquid is also a variable in the cavitation conditions of the irrigant, as such a low viscosity fluid could be selected as the irrigant. The low surface tension and/or low viscosity fluid could be an irrigant selected with such properties or could be an irrigant with an additive which reduces its properties.

The irrigant may also be selected or tailored to increase its abrasive impact. For example, the density of the irrigant may be increased and/or the irrigant fluid may further comprises abrasive particles (for example solid particles suspended in the fluid).

The needle assembly in accordance with embodiments may use a needle having a forward-facing axial opening. Such forward opening ended needles typically produce a better forward flow of irrigant but, in contrast to embodiments of the invention, many prior art configurations avoid the use of such needles due to the risk of pushing aggressive irrigant such as NaOCl beyond the apex of the tooth and causing significant pain and severe complications.

Implementations of the embodiments may include a method of endodontic irrigation, the method comprising: positioning a needle of the present needle assembly within a portion of the root canal; supplying irrigant fluid to the needle under pressure such that fluid is expelled from the needle tip into the root canal; and selecting the delivery pressure of the irrigant fluid such that the pressure at the needle tip exceeds a threshold cavitation pressure such that the flow of irrigant through the needle causes a cloud of inertial cavitation to be formed within the irrigant fluid in the root canal forward of the needle tip.

The delivery pressure may be selected from a range of between 5 and 300 bar. In particular, the delivery pressure may be selected from a range of between 5 and 100 bar.

The method may further comprise heating the irrigant fluid. For example, the irrigant fluid may be heated to between 20 to 57° C.

The method may further comprise adjusting the pressure depending upon the depth the needle is inserted into the canal.

Some embodiments may comprise pulsing the supply of irrigant fluid. A pulsed (rather than continuous flow) allows a burst of flow to recoil on impact and result in an increased impulse (rate of change of momentum) in the irrigant. This increases the erosion potential of the flow and therefore further increases the debridement and/or disinfection efficiency. A pulsed flow may also provide the advantage of reducing the overall quantity of irrigant used.

Implementations of the embodiments may include a dental apparatus comprising: a supply of fluid; a pump for delivering fluid from the supply under pressure; a handpiece in fluid communication with the pump and comprising a needle, extending from a rearward end proximal to the handpiece to a forward tip distal from the handpiece, a lumen of the needle extending to an opening at the tip to deliver fluid received from the pump to a tooth; and wherein the dimensions of the needle and the delivery pressure of the fluid are selected such that the flow of fluid through the needle causes a cloud of inertial cavitation to be formed forward of the needle tip.

The needle dimensions may, for example, be selected depending upon the procedure required. For example, the procedure may include one or more of debridement and/or disinfection of a cavity, dental plaque removal from the outside of the tooth or at subgingival surfaces, drilling through dental tissue (dentine, enamel), cutting of soft tissue, or finding entrances to root canals. The length of the needle may be selected to ensure appropriate placement of the needle tip in use. The dimensions of the needle may be selected to ensure that a sufficient flow is possible through the needle whilst also ensuring that the needle tip can be suitably located. The dimensions may also be selected to ensure that a backflow of fluid is formed proximal to the flow of fluid ejected from the needle.

Unless otherwise stated, each of the integers described may be used in combination with any other integer as would be understood by the person skilled in the art.

Further, although all aspects of the invention preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

Further, in the discussion of the invention, unless stated to the contrary, the disclosure of alternative values for the upper or lower limit of the permitted range of a parameter, is to be construed as an implied statement that each intermediate value of said parameter, lying between the smaller and greater of the alternatives, is itself also disclosed as a possible value for the parameter.

In addition, unless otherwise stated, all numerical values appearing in this application are to be understood as being modified by the term "about".

Whilst the invention has been described above, it extends to any inventive combination of the features set out above or in the following description or drawings.

### Description of the Drawings

Embodiments of the invention may be performed in various ways, and embodiments thereof will now be described by way of example only, reference being made to the accompanying drawings, in which:
**Figure 1** shows a schematic of an apparatus in accordance with an embodiment of the invention;
**Figure 2** shows a detail of the schematic of figure 1 showing the needle position within a tooth; and
**Figures 3(A), 3(B), 3(C) and 3(D)** represent the working principle behind embodiments of the invention.

### Detail Description of Embodiments

It may be noted that proximal and distal are used herein to conveniently refer to the device in its typical in use orientation. Thus, it will be understood that proximal will generally mean a surface, component or direction which is proximal to the operator's hand during use and distal may be used to generally mean the surface, component, or direction distal to the operator's hand (and which will therefore be proximal to the root canal). Forward will likewise be understood to be used with respect to the directions away from the proximal end and towards the distal end (and rearwards understood to be the reverse direction). However, it will be appreciated that such references are not intended to be limiting and that the device may take any orientation in use.

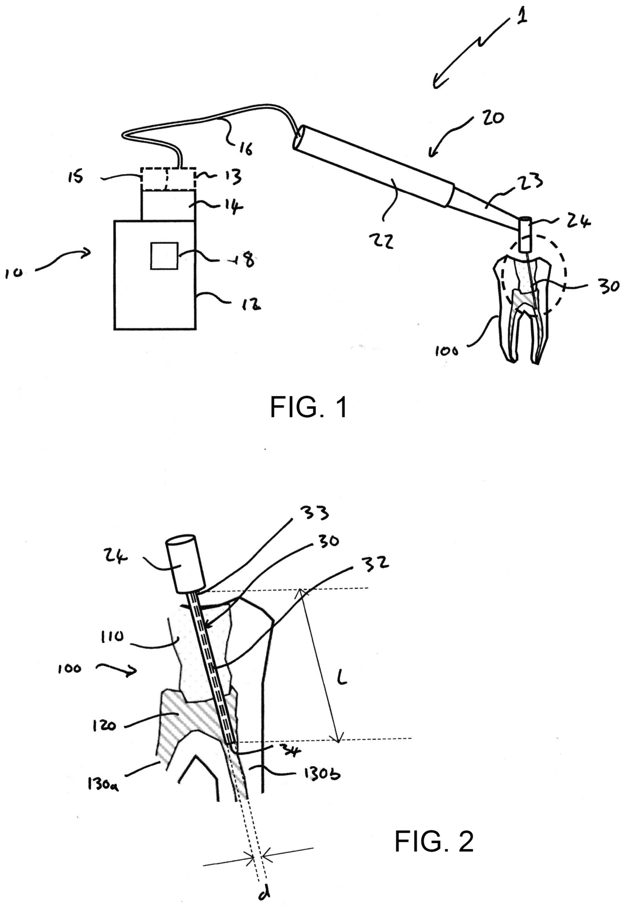

An endodontic irrigation apparatus 1 is shown schematically in figure 1. The apparatus comprises a base unit 10 including a reservoir 12 containing a supply of irrigant fluid and a pump 14 for delivering irrigant fluid from the supply under pressure through the flexible conduit 16. The base unit 10 may also include a user interface 18 (which may be of any convenient format) and may enable an operator to adjust operating parameters such as the pressure output of the pump 14.

A handpiece 20 is connected to the distal end of the flexible conduit 16, for example by a conventional removable connector, such that the handpiece 20 is in fluid communication with the base unit 10 and can receive irrigant fluid from the supply 12 via pump 14. The handpiece 20 includes a grip portion 22 at a proximal end and a head 24 connected via a neck 23 to the grip portion 22. The head 24 extends to a needle 30 which may typically be replaceably mounted into the head 24. It may be appreciated that as used herein the term "needle" refers broadly to a thin elongate conduit having a bore (the "lumen" of the needle) extending therethrough and extending from a proximal end for receiving a supply of fluid in use to an opening at a distal end for delivering fluid in use. As best seen in figure 2, the needle extends axially from a proximal end 33 to a distal tip end 34 and has a length I. A lumen 32 extends through the length of the needle 30 to provide a passageway for irrigant. The tip of the needle 34 ends with a forward-facing axial opening such that the irrigant is expelled in a forward axial stream from the lumen.

In use the needle 30 is inserted into a tooth 100 via a cavity 110 (formed by any convenient manner for example by drilling) which provides access to the pulp chamber 120. In accordance with embodiments of the invention the needle has a length, measured in direction I, of at least 5mm and an external diameter, measured in direction d, of no more than 520 µm such that the needle tip 34 is able to be positioned within a portion of the root canal 130. With the needle positioned in such a manner the applicant has surprisingly found that provided the irrigant is supplied in a manner which creates a cloud of inertial cavitation forward of the needle tip 34 it will provide effective debridement and/or disinfection without the need for an NaOCl based irrigant. In contrast, many prior art systems use a needle which is either too short to reach the root canal itself (and instead merely position the tip in the cavity 100 or pulp chamber 120) and/or which is of too great a diameter to enter the root canal.

Further, embodiments of the invention enable root canal treatment to be carried out without the need for mechanical filing (at least in all but the most difficult of cases - for example older patients where canals become calcified and narrowed) such that prior to the use of the apparatus of the invention only the initial access to the root canal need be made. In order to provide such a cloud of inertial cavitation the applicant has found that the internal diameter of the needle (i.e. the diameter of the lumen) and delivery pressure should be selected (dependent upon the geometry of the specific tooth and needle combination) which is in excess of a threshold pressure cavitation pressure such that the flow of irrigant through the needle causes a cloud of inertial cavitation to be formed within the irrigant fluid in the root canal forward of the needle tip. For example, the lumen diameter could be at least 25µm, for example at least 50µm. Without understanding of this effect it may be natural to select a needle having too small an internal diameter (for example less than 50 µm in order to ensure that it fits into the root canal), but the applicant recognises that it is important that such a needle will provide frictional loses which mean that even a very high delivery pressure will not provide a flow leaving the needle which creates effective cavitation. In contrast in embodiments of the invention the cavitation providesstrong debridement, disinfection and/ or removal of debris or bacteria due to the well-known erosive effect which results from the shockwaves caused by rapidly collapsing vapour bubbles within the fluid.

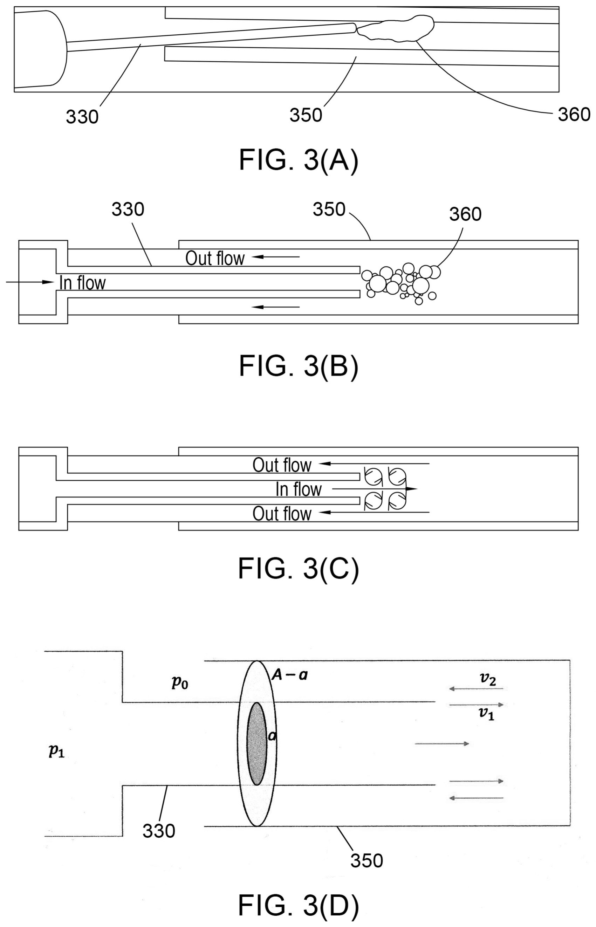

As shown in the 16,000-fps high-speed photograph of figure 3(A) glass micropipettes (having for example inner diameters of 1.2 or 0.6mm) can be used to simulate the root canal. When an appropriately sized needle 330 is inserted into the tube 350 and a flow provided at a pressure exceeding the cavitation threshold a cavitation cloud 360 is clearly formed downstream of the needle. The flow within the tube 350 is illustrated schematically in figure 3(B). Importantly, the size of the needle (no more than 520 µm) ensures that the flow of fluid in the tube (or root canal in practice) includes both an inflow from the needle and an outflow passing between the walls of the tube and the exterior of the needle.

Cavitation occurs under the right conditions when a liquid is transformed rapidly into a gas across the phase boundary. Without being bound by specific theory, the applicant has recognised that, as illustrated in figure 3(C), the selection of a needle which enable a backflow to be generated in the root canal causes a strong shear layer effect to form between the inward and outward flow. This shear layer increased vortices in the flow and significantly increases the occurrence of cavitation. The resulting conditions imply that very strong vortices can be generated at the interface between in and out flow inside the root canal. Inside the vortex, the (dynamic) pressure would be strongly reduced. The reduction in pressure makes cavitation more favourable (bringing the starting point closer to the phase boundary). The result of this effect is that a cavitation cloud is produced within a passage such as a root canal at flow conditions (pressure, speed and flow rate) which would not create cavitation in an open environment. Increasing the speed at which the liquid exists the needle will also aid in increasing the vortices and for a given channel, there will exist a minimum nozzle exit velocity below which cavitation will not occur. Provided the internal diameter of the needle is not overly narrow, the delivery pressure may be used to control the needle exit velocity.

To test the performance of a device in accordance with embodiments, tests were performed on transparent plastic teeth (RepliDens mandibular molar, transparent type 03.2.1, Medcem GmbH, Weinfelden, Switzerland) having a realistic root canal structure filled with coloured gelatine which is used to simulate the tissue inside the tooth. Different devices were tested and the amount of gelatine before and after cleaning was measured using image analysis and pixel counting. The apparatus in accordance with an embodiment used a needle of 20mm length and 30G gauge (corresponding to an internal diameter of 0.16mm and an external diameter of 0.31mm). The delivery pressure was set to 60 bar. The irrigant was saline solution and the needle was positioned and moved up and down the canal for 180 seconds. The results of multiple root canal systems were compared based upon the quantity of gelatine before and after cleaning to determine the % of material removed. The same test was performed using commercial ultrasonic and laser-based irrigant activation systems. In the case of the ultrasonic (EDDY, VDW GmbH, Munich, Germany), the vibrating tips were inserted into each canal and activated for 120 seconds. For the laser system (LiteTouch Er:YAG Laser, Orcos Medical AG, Küsnacht, Switzerland), plastic teeth pulp chamber was filled with water into which the laser tip was placed and activated for 120 seconds. The results are shown in Table 1 below with the embodiment of the invention providing significantly improved debridement in uninstrumented teeth (our invention) than commercially available commercial systems (ultrasonic system (without instrumenting/filing), Laser system (without instrumenting/filing), and instrumented mechanical filing (ProTaper, Dentsply, Ballaigues, Switzerland) followed by syringe irrigation. The experiment found that conventional ultrasonic and laser activation systems failed to adequately remove materials from inside the canals. They thus can be used for activation only, are not fit to treat uninstrumented canals, and cannot reduce the need for mechanical filing. In the case of the ultrasonic system, the tip could not vibrate side to side due to the narrow root canals - dampening the oscillations. In the case of the laser system, we observed that no gelatine would come out of the root canals as not enough flow was generated. Mechanical files together with flushing using a syringe filled with water performed better but was less effective that embodiments of the invention and significantly more time consuming.

**Table 1: cleaning efficacies of different systems in uninstrumented tooth models**

| | Cleaned area [%] | Standard deviation |
|---|---|---|
| Ultrasonic | 59.7 | 10.3 |
| Laser system | 80.9 | - |
| Mechanical filing | 89.8 | 8.1 |
| Our invention | 97.1 | 1.0 |

Importantly, the applicant has also compared the conditions between an open ended and closed/confined area (with the tooth root in being closed). This has demonstrated unexpected results and illustrates the importance of the geometry of the tooth canal and needle on cavitation. It is believed that prior systems have failed to consider this as a factor, and this reflects why such system may fail to provide true effective cavitation.

To demonstrate this effect, we performed an experiment using a delivery pressure of 60 bar connected to needles of different shapes, diameters, and lengths. The water coming out of the needles was ejected into either a (i) bath of water, (ii) a glass micropipette with an open end, or (iii) a glass micropipette completely sealed at one end. The needles tested included needles of standard gauge sizes. The threshold pressure was recorded as the point at which a stable cloud of cavitation was first visible. The threshold for the upstream pressure to generate developed cavitation was generally much lower inside a closed end, narrow canal compared to a free water bath. From the experimental data it was possible to note that 30G (needle gauge) needle with 20mm or 15 mm only generated cavitation inside the micropipette and not in open bath, to generate cavitation in an open bath higher pressure would be required. All other needle sizes including 30G needles with a 10 mm or 5 mm length, cavitate in open water. However, using them inside the micropipettes reduce the needed upstream pressure by 15 bar to 40 bar. An exception was found for the 25G needle and the 0.6mm tube (Table 2; Closed-end micropipette d=0.6mm, 25G) - in this case the needle blocked the backflow itself and therefore cavitation threshold increased after closing the micropipette end (because the external diameter of the needle was very close to the internal diameter of the pipette tube). Thus, the applicant has been able to confirm that the backflow within the passage is required to induce cavitation efficiently.

**Table 2: Cavitation Pressure for various needle configurations**

| | | | **Cavitation pressure threshold [bar]** | | | | |
|---|---|---|---|---|---|---|---|
| **Need le type** | Needle exit diamet er [mm] | Needl e lengt h [mm] | Free in wat er bath | Open-end micropipe tte d=1.2mm | Closed-end micropipe tte d=1.2mm | Open-end micropipe tte d=0.6mm | Closed-end micropipe tte d=0.6mm |
| 30G | 0.159 | 20 | - | - | 80 | 80 | 60 |
| | | 15 | - | - | 60 | 70 | 50 |
| | | 10 | 70 | 50 | 45 | 50 | 30 |
| | | 5 | 70 | 65 | 45 | 50 | 30 |
| 25G | 0.260 | 20 | 70 | 50 | 20 | 20 | - |
| | | 15 | 60 | 50 | 12 | 20 | 70 |
| | | 10 | 50 | 50 | 6 | 15 | 60 |
| | | 5 | 50 | 50 | 3 | 15 | 50 |

The volume flow through the needle is strongly dependent on the upstream pressure and needle diameter. As such when a lower pressure is needed to generate cavitation the volume of the flow is reduced. This is advantageous in practice as lowering the rate of flow and/or the pressure can reduce the risk that the jet induces any unwanted damage in the tooth due to the high flow rate. The experiments found that the highest volume flow was for the biggest needle diameter 25G and lowest flow for smallest diameter 34G. The lowest pressure threshold for cavitation occurred with a 25G needle having a length of 10 mm - this was 6 bar with 72 ml/min. These results showed that inside narrow closed end canals, the threshold for cavitation drops significantly. Thus, embodiments of the invention may generate effective cavitation at lower upstream pressure with a lower volume flow. Such flows provide significant advantages in reduced pressure at the root canal apex and lower volume flow which both minimise the risk of apical extrusion.

Thus, the results confirmed that the properties of the needle (such as diameter, and length) have a significant influence on the threshold cavitation pressure. Furthermore, experiments confirmed that the effect of the backflowing fluid is very important - increasing the relative velocity and the building of vortices, thereby significantly decreases the required pressure to generate cavitation.

The effect of needle length on the cavitation threshold is simple. A longer needle increases the pressure required for cavitation which is considered to be consistent with the fact that a shorter needle will provide less flow resistance. However, in practical embodiments this will generally mean that the selection of needle length is a compromise between the increase in threshold pressure and the length required to position the tip sufficiently within the root canal to deliver the cavitation and efficiently debride the canal.

Although the invention has been described above with reference to preferred embodiments, it will be appreciated that various changes or modification may be made without departing from the scope of the invention as defined in the appended claims.

For example, some embodiments of the invention may include a heater 15 to increase the temperature of the irrigant (thus moving it closer to the phase boundary for a given pressure and further making cavitation more favourable). The heater 15 may be included as part of the base unit 10 or may be integrated into the handpiece. In some embodiments the pump 14 or base unit may include a pressure regulator.

In addition, or as an alternative to, the user interface 18 the handpiece 20 may include controls such as a switch on the handpiece (or associated with the handpiece for example on a foot pedal). For example, a trigger may be provided for activation of flow through the system.

As embodiments of the invention enable the use of a simple irrigant such as water or saline, it may be appreciated that embodiments may provide for a variety of options in use. For example, the irrigant could be a low-surface tension liquid or a high viscosity liquid. The irrigant could also include additions such as abrasive particles.

In some embodiments the apparatus may include canal sensing system. For example, to ensure liquid does not go past the tooth apex, embodiments may include an apex locator to measure the distance to the apex and help the dentist to operate the device.

Whilst the primary purpose of the endodontic irrigation apparatus of embodiments may be root canal procedures, it may also be appreciated that the debridement and/or disinfection effect of the cavitation stream could also be applied to other uses within a dental practice. For example, the device could be used to for dental plaque removal from the outside of the tooth or at subgingival surfaces. Embodiments could also be used to drill through dental tissue (dentine, enamel) or for cutting of soft tissue. The apparatus could also be utilised to find entrances to root canals.

## Claims

1. A needle assembly for an endodontic apparatus, comprising:
a head (24) for removably coupling the needle assembly to a handpiece (20); and
a needle (30) extending from the head (24) at a proximal end (33) to a distal tip (34), a lumen (32) of the needle extending to an opening at the tip (34) to deliver fluid into a root canal, the opening at the tip (34) of the needle (30) being a forward-facing axial opening such that the fluid is expelled in a forward axial stream from the lumen (32); wherein
at least the distal end of the needle (30) has a needle gauge size between 30G and 34G, such that the needle tip is positionable within a portion of the root canal to enable a backflow to pass between the root canal and the needle (30); and
the lumen (32) has a diameter at the tip of at least 50 µm to enable fluid to be delivered at a delivery pressure in excess of a threshold cavitation pressure, wherein delivery of fluid through the needle (30) at a delivery pressure in excess of a threshold cavitation pressure into the root canal generates a backflow in the root canal that facilitates forming of a cloud of inertial cavitation within the fluid in the root canal forward of the needle tip (34).

2. The needle assembly of claim 1, wherein the needle (30) has a length, extending from its rearward end (33) to its tip (34), of at least 3mm.

3. The needle assembly of any preceding claim, wherein the needle length is between 10 and 30mm.

4. The needle assembly of claim 1 or 2, wherein the needle (30) has an external diameter at the tip (34) of no more than 520 µm.

5. The needle assembly of any preceding claim, wherein the fluid is delivered at a delivery pressure between 5 and 300 bar.

6. The needle assembly of any preceding claim, wherein the fluid is delivered at a delivery pressure selected such that the minimum exit velocity of the irrigant at the needle tip is at least 20 m/s.

7. The needle assembly of any preceding claim, wherein the needle (30) comprises a distal portion including the open forward tip (34) and a proximal portion connected to the proximal end (33) of the distal portion, and wherein the proximal portion has an increased diameter.
